# EUROPEAN PATENT APPLICATION

(11) **EP 4 616 832 A1**
(43) Date of publication of application: **17.09.2025**
(21) Application number: 24162950.0
(22) Date of filing: 12.03.2024
(51) Int. Cl.: A61F 13/475, A61F 13/532, A61F 13/535, A61F 13/534

(54) **ABSORBENT ARTICLE WITH PROFILED LATERAL ABSORBENT ZONES**

(71) Applicant: The Procter & Gamble Company, Cincinnati, OH 45202 (US)
(72) Inventor: Garcia, Julien René, 65824 Schwalbach am Taunus (DE)
(74) Representative: P&G Patent Germany

(57) **Abstract**

Absorbent article (20) for personal hygiene such as diaper comprising an absorbent core (28) having two lateral absorbent zones (61, 63) disposed between respectively between a first and second longitudinally-extending channels (26a, 26b) and a first and second longitudinal edges (604, 606) of an absorbent layer (60). The lateral absorbent zones are profiled in the transversal direction, so that the basis weight of the absorbent material is higher towards the respective channel than towards the respective longitudinal side edge.

## Description

### FIELD OF THE INVENTION

The invention relates to personal hygiene absorbent articles such as, but not limited to, baby diapers and adult incontinence products. The absorbent core of the invention comprises an absorbent layer with channels and has an improved profiling of absorbent material in the lateral absorbent zones between the channels and the longitudinal side edges of the absorbent layer.

### BACKGROUND OF THE INVENTION

Absorbent articles for personal hygiene such as diapers are designed to absorb and contain body exudates, in particular large quantity of urine. These absorbent articles comprise several layers providing different functions, such as a topsheet, a backsheet and in-between an absorbent core, among other layers. The absorbent core absorbs and retains exudates in order to keep the wearer's skin dry and avoid soiling of clothes or bedsheets. It is desirable that the absorbent core should make the most efficient use possible of the absorbent material to save material costs and keep the diapers as thin as possible.

The majority of currently marketed absorbent articles comprise a blend of cellulose fibers with superabsorbent polymers (SAP) particles as absorbent material), see for example US5,151,092 (Buell). Absorbent articles having a core consisting essentially of SAP without cellulose fibers as absorbent material (referred in the art as "airfelt-free" cores) have been more recently proposed. For example WO2008/155699 (Hundorf et al.) discloses absorbent cores with a patterned layer of SAP immobilized by a net of fibrous thermoplastic adhesive material deposited over the layer of SAP. The fibrous adhesive net immobilizes the SAP in position within the absorbent core without substantially restricting the ability of the SAP to absorb large volumes of urine. WO2012/170783 (Hundorf et al.) discloses absorbent cores comprising absorbent material having a basis weight that varies across the absorbent core. WO2012/170778A1 (Rosati et al., see also WO2012/170779, WO2012/170781 and WO2012/170808) WO2012170778A1 discloses absorbent structures that comprise superabsorbent polymers, optionally a cellulosic material, and at least a pair of substantially longitudinally-extending channels. The core wrap can be adhesively bonded through the channels to form a channel bond. The integrity of the channel bonds may be at least partially maintained in the wet state. EP3,238,677 (Bianchi et al.) further discloses that the basis weight of the central absorbent zone between the first and the second channels may be lower than the average basis weight between first and second lateral absorbent zones disposed laterally outwardly of the channels.

While the absorbent cores of the prior art generally have good properties, there is a continuous need to improve comfort, fit and efficiency of the absorbent cores while improving or at least maintaining key properties such as speed of acquisition and retention of fluid. In addition, it is desirable to reduce raw material usage, in particular SAP for cost and sustainability reasons. The present invention addresses all these problems.

### SUMMARY OF THE INVENTION

The invention is directed to absorbent article for personal hygiene as indicated in the claims. The absorbent article extends in a plane formed by a longitudinal centerline extending in a longitudinal direction and a transversal centerline extending in a transversal direction perpendicular to the longitudinal direction. The absorbent article comprises a topsheet, a backsheet, an absorbent core between the topsheet and the backsheet, and optionally an acquisition layer between the topsheet and the absorbent core. The absorbent core comprises a core wrap comprising a top side and a bottom side, and an absorbent layer between the top side and the bottom side of the core wrap. The absorbent material forming the absorbent layer comprises superabsorbent polymer particles optionally mixed with cellulose fibers. The absorbent layer has a periphery, as seen from above in a plane parallel to the longitudinal and transversal directions, comprising a front edge, a back edge, a first longitudinal side edge on one side of the longitudinal centerline and a second longitudinal side edge on the other side of the longitudinal centerline. The absorbent core comprises at least one longitudinally-extending channel, the channel having a length measured in the longitudinal direction, the channel being substantially free of absorbent material, and wherein the top side of the core wrap is attached to the bottom side of the core wrap through the channel. The absorbent core comprises a first lateral absorbent zone and a second lateral absorbent zone extending respectively transversally from a first channel to the first longitudinal edge, and from a second channel to the second longitudinal edge (the first channel and second channel can be at least partially the same channel, in particular if the absorbent core comprises only one central channel, or the first and second channels may be entirely or partially distinct channels, for example when the core comprises two partially joined or completely distinct symmetrically-disposed channels relative to the longitudinal centerline).

The first and second lateral absorbent zones are profiled in the transversal direction so that the basis weight of the absorbent material in each lateral absorbent zone is higher towards the respective channel than towards the respective longitudinal side edge. In a first aspect, this is characterized in that the first lateral absorbent zone and the second lateral absorbent zone each has a Lateral Absorbent Zone Profile Ratio of at least 1.40, as measured according to the Lateral Absorbent Zone Profile Ratio Test described herein.

The first and second channels may be advantageously at least partially disposed in the crotch portion of the article.

The first and second channels may each have a length as measured in the longitudinal direction which is at least 10 cm.

The absorbent core may comprise a first longitudinally-extending channel and a second longitudinally-extending channels which are symmetrically disposed on each side of the longitudinal centerline. The channels may be completely or at least partially distinct, so that the absorbent core comprises a central absorbent zone between the channels.

The absorbent material may be free of cellulose fibers. Alternatively, the absorbent material may comprise superabsorbent polymer particles mixed with cellulose fibers as absorbent material, and the first longitudinal side edge and the second longitudinal side edge of the absorbent layer is straight and parallel to the longitudinal direction.

The invention also covers a package comprising a plurality of absorbent articles as described above and in the claims. The invention also covers the absorbent core per se as described above and in the claims.

Further possible and advantageous features are further described in the attached claims.

These and further aspects will now be further described in the following description.

### BRIEF DESCRIPTION OF THE DRAWINGS

Fig. 1 is a top view of an absorbent article in the form of a taped diaper with an absorbent core comprising two curved channels, with some layers partially removed;
Fig. 2 is a schematic transversal cross-section of the core of Fig. 1;
Fig. 3 is a schematic illustration of the absorbent core when used on a wearer;
Fig. 4 is a top view of the absorbent core used in the diaper of Fig. 1, with the top core wrap layer partially removed;
Fig. 5 shows the distribution of absorbent material in a comparative absorbent core;
Fig. 6 shows the distribution of absorbent material in an absorbent core of the invention;
Fig. 7 schematically compares the distribution of absorbent material between a comparative core and an exemplary core in the transversal direction (CD direction);
Fig. 8 shows the inner region and outer region of the lateral absorbent zones for conducting the Lateral Absorbent Zone Profile Ratio Test;
Figs. 9-13 show inner region and outer region for alternative channel shapes.

### DETAILED DESCRIPTION OF THE INVENTION

### General description of the absorbent article 20

As used herein, "absorbent articles" refers to personal hygiene devices that are placed on the crotch of a wearer to absorb and contain body exudates. Baby care articles are products intended for babies, toddlers and/or children, relating to disposable absorbent articles including taped diapers, pant diapers, absorbent inserts. Feminine care articles are products relating to catamenial pads, incontinence pads, interlabial pads, panty liners, pessaries, sanitary napkins. Adult incontinence articles are products intended for adults, relating to disposable absorbent articles including taped and pant diapers, absorbent inserts, incontinence pads, panty liners. While the invention is especially useful for baby care articles, it may also be used in feminine care articles or adult incontinence articles. The absorbent articles of the invention are typically disposable and are preferably recyclable.

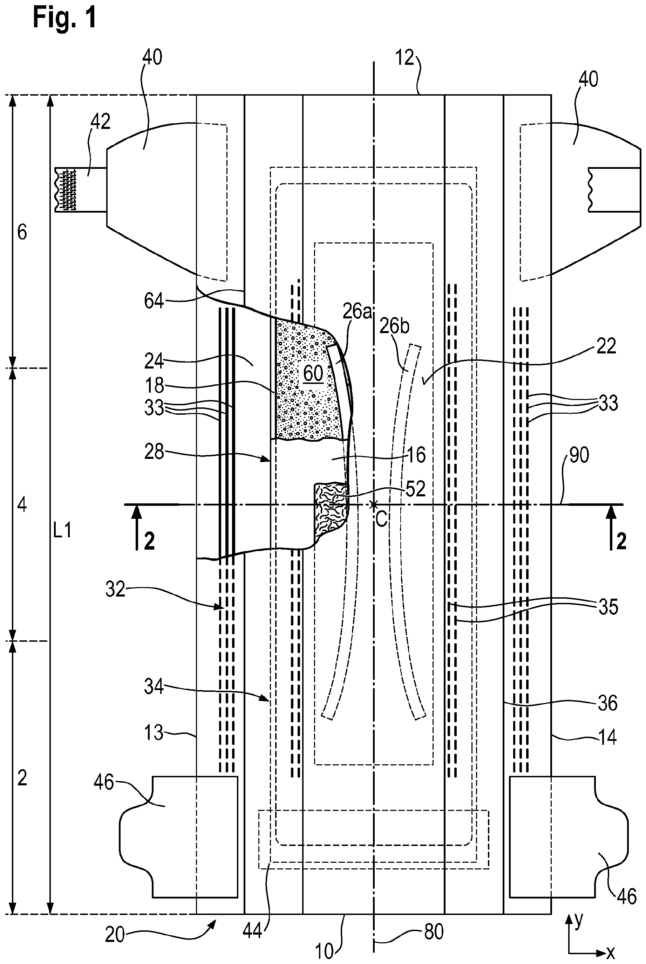

As used herein, "diapers" refers to absorbent articles generally worn by babies, infants and incontinent adults about the lower torso so as to encircle the waist and legs of the wearer and that is specifically adapted to receive and contain urinary and fecal waste. Diapers are typically proposed as taped diapers or pant diapers. Taped diapers have a fastening system (as illustrated in Fig. 1 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

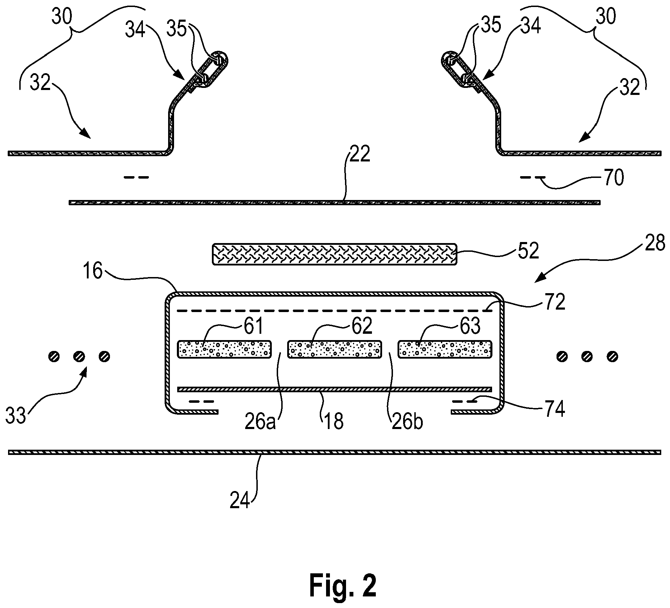

An exemplary absorbent article according to the invention in the form of a baby taped diaper 20 is represented in Figs. 1-2. Fig. 1 is a top plan view of the exemplary diaper 20, in a flat-out state, with portions of the structure being cut-away to show the construction of the diaper more clearly. Fig. 2 is transversal cross-sectional view of the diaper 20 taken along line 2-2 in Fig. 1. This diaper 20 is shown for illustration purpose only, as the invention may be used for making a wide variety of diapers or other absorbent articles such as training pants, adult incontinence pants or feminine sanitary pads. For ease of discussion, the articles and absorbent cores of the invention will be discussed with reference to the Figures and the numerals referred to in these Figures, however these are not intended to limit the scope of the claims unless specifically indicated.

The absorbent article 20 comprises a liquid permeable topsheet 22, a liquid impermeable backsheet 24 and an absorbent core 28 according to the invention between the topsheet and the backsheet. The absorbent article may also comprise further typical components such as an acquisition layer 52 (some absorbent articles, especially with an airfelt-free core, comprises a dual layer acquisition-distribution system, for simplicity only an acquisition layer 52 is represented in the Figures). Elasticized gasketing cuffs 32, also referred to as outer cuffs, and upstanding barrier leg cuffs 34, sometimes referred to as inner cuffs, are further detailed in the following. Figure 1 also shows other typical taped diaper components such as a fastening system comprising fastening tabs 42 attached to back ears 40 in the back region towards the back edge 12 of the article and cooperating with a landing zone 44 towards the front edge 10 of the article. The absorbent article may also comprise other typical components, which are not represented in the Figures, such as a back elastic waist feature, a front elastic waist feature, transverse barrier cuffs, a lotion application, etc.

The absorbent article 20 comprises a front edge 10, a back edge 12, and two longitudinally-extending side (lateral) edges 13, 14. The front edge 10 is the edge of the article which is intended to be placed towards the front of the user when worn, and the back edge 12 is the opposite edge. The absorbent article can be notionally divided by a longitudinal centerline 80 extending from the front edge to the back edge of the article and dividing the article in two substantially symmetrical halves relative to this centerline, when viewing the article from the wearer facing side in a flat-out configuration, as exemplarily shown in Fig. 1. The article's longitudinal centerline 80 is typically aligned with the longitudinal centerline 80 of the core, and these are referred to as the same numeral in the Figures. If some part of the article is under tension due to elasticized components, the article may be typically flattened along the periphery of the article using clamps and/or a sticky surface, so that the topsheet and backsheet can be pulled taut to be substantially flat. Closed articles such as training pant may be cut open along the side seams to apply them on a flat surface. Unless otherwise indicated, dimensions and areas disclosed herein apply to the article in this flat-out configuration.

The article has a length L as measured along the longitudinal centerline 80 from the middle of the back edge to the middle of the front edge. The absorbent article 20 also comprises a transversal centerline 90 perpendicular to the longitudinal centerline and passing through the point C disposed on the longitudinal centerline at an equal distance between the front edge and the back edge of the article. Contrary to the longitudinal centerline, the article is typically not generally symmetrical relative to the transversal centerline. The article may be further notionally divided in a front region 2, a crotch region 4 and a back region 6, each of these regions having one third of the length L of the article. The front region extends from the front edge 10 of the article inwardly to the crotch region 4, the crotch region 4 is disposed between the front region 2 and the back region 6, and the back region 6 extends from the back edge 12 inwardly to the crotch region 6.

The longitudinal centerline 80 extends in a longitudinal direction (y) and the transversal centerline 90 in a transversal direction (x). The absorbent article and the core can be represented flat in a plane defined by the (x) and (y) directions.

The topsheet 22, the backsheet 24, the absorbent core 28 and the other article components may be assembled in a variety of well-known configurations, in particular by gluing and/or heat embossing. Exemplary diaper assemblies are for example generally described in US3,860,003, US5,221,274, US5,554,145, US5,569,234, US5,580,411, and US6,004,306.

Typically, when dry, the thickness of the article is much smaller than its length and width. The absorbent article is preferably thin. The caliper (thickness) of the article may advantageously ranges from 1.0 mm to 8.0 mm, in particular from 1.5 mm to 6.0 mm, as measured at the intersection C of the longitudinal centerline and transversal centerline, using the Absorbent Article Caliper Test described below.

### General description of the absorbent core 28

As used herein, the term "absorbent core" or "core" refers to a component which is placed or is intended to be placed within an absorbent article. The absorbent core comprises an absorbent material disposed in a core wrap. The absorbent core has typically the most absorbent capacity of all the components of the absorbent article and comprises all or at least the majority of superabsorbent polymer (SAP). The core typically thus consists essentially of, or consists of, the core wrap, the absorbent material and typically some amount of adhesive to maintain the integrity of the core. The absorbent material may comprise or consist of SAP particles as exemplified in the present description, but other absorbent materials may be used, in particular a mixture of cellulose fibers and SAP, as is known in the art. The terms "absorbent core" and "core" are herein used interchangeably.

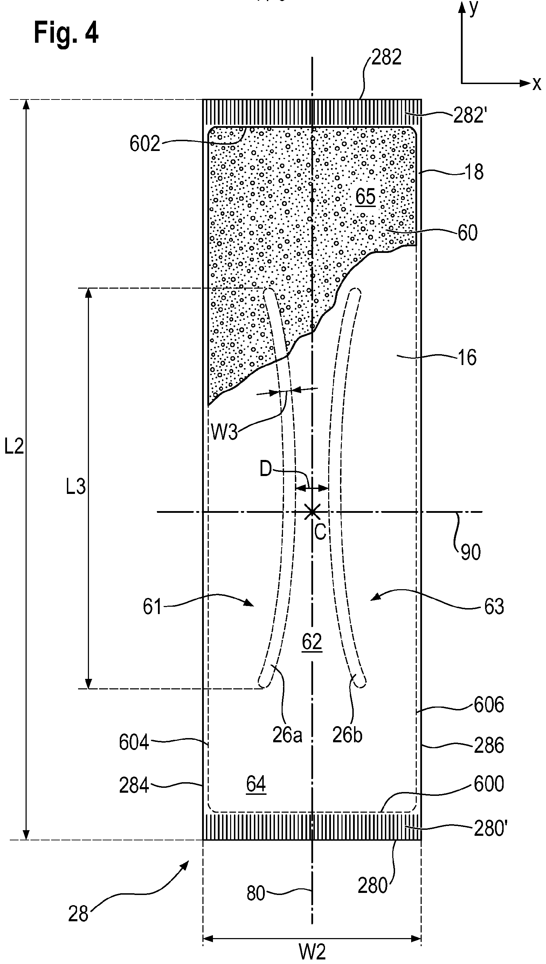

The absorbent core typically extends in a longitudinal direction and a transversal direction as defined above, and the absorbent core may be represented flat in a plane parallel to these direction as shown in Fig. 4. The absorbent core may also be typically thin and conformable, so that it can be laid on a curved surface for example a drum during its making process or stored as a continuous roll of stock material before being converted into an absorbent article. For ease of discussion, the exemplarily absorbent core of Fig. 4 is represented in a flat state and extending in a plane along the transversal direction (x) and the longitudinal direction (y). Unless otherwise indicated, dimensions and areas disclosed herein apply to the core in this flat-out configuration. The same applies to the absorbent article, as exemplarily represented in Fig. 1, in which the core is comprised.

The outline of the absorbent core is typically defined by the core wrap. The core wrap comprises a top side 16 and a bottom side 18, which may be formed by two individual substrates (not separately numbered in the drawings) forming respectively the majority of the top side 16 and bottom side 18. The two substrates may be disposed for example in a C-wrap configuration as illustrated in Figs. 1-4. It is also common and possible to have a single substrate forming the top side and the bottom side of the core wrap. The absorbent core typically comprises a front edge 280, a back edge 282 and two longitudinally-extending side edges 284, 286 joining the front edge and the back edge. The front edge is the edge of the core intended to be placed towards the front edge 10 of the absorbent article in which the core is or will be integrated. Typically the absorbent material 60 of the core may be profiled in the longitudinal direction, so that there is higher absorbent material amount towards the front edge than towards the back edge, as more absorbency is typically required towards the front half of the article. Typically the front and back edges 280, 282 are shorter than the longitudinally-extending side edges 284, 286. The top side 16 of the core is the side placed or intended to be placed towards the topsheet 22 of the article and the bottom side 18 is the side placed or intended to be placed towards the backsheet 24 in the finished article. The top side of the core wrap may be typically treated to be more hydrophilic than the bottom side. The absorbent core may have a profiled distribution of material in the longitudinal direction, especially having a higher basis weight in the crotch region than in the front region, and still higher in the front region than in the back region.

The absorbent core can notionally (i.e. virtually) be divided in two halves by a longitudinal centerline 80 extending from the middle of the front edge 280 to the middle of the back edge 282. The core is typically symmetrically constructed relative to this centerline, when viewing the core in the plane formed by the transversal and longitudinal direction (x, y). The longitudinal centerline of the core is substantially aligned with the longitudinal centerline of the absorbent article, notwithstanding normal process variation in the transversal direction that may somewhat shift the absorbent core or other components slightly off the ideal alignment.

The absorbent core may be generally rectangular with a width W2 in the transversal direction and a length L2 in the longitudinal direction as measured from edge to edge, including the region of the core wrap which does not enclose the absorbent material, in particular at the front and back end seals 280', 282', when such seals are present. If the core is not rectangular, the maximum dimension measured along the transversal direction and the longitudinal direction can be used to report the width and length of the core respectively. The width and length of the core may vary depending on the intended usage. For baby and infant diapers, the width W2 may for example in the range from 40 mm to 200 mm and the length L2 from 100 mm to 600 mm. Adult incontinence products may have higher maximum dimensions.

The transversal centerline 90 of the article and the centerpoint C as defined above for the absorbent article can be reported on the absorbent core as illustrated in Fig. 4. The caliper of the core (dry, i.e. before use) as measured at the center point (C) or at any other points of the surface of the core according to the Dry Core Caliper Test as described herein may be from 0.25 mm to 5.0 mm, in particular from 0.5 mm to 4.0 mm.

The absorbent material forming the absorbent layer 60 may be any conventional absorbent material used in absorbent articles. An auxiliary glue 72 may be present to at least partially immobilize the absorbent material. Typical absorbent material are cellulose fibers/ SAP particles mix. The SAP proportion may range from 50% to 90%, in particular from 60% to 80% by weight of the absorbent material. Alternatively, the absorbent material may consist of SAP particles and be free of cellulose fibers. The SAP particles may then be immobilized by the auxiliary glue 72 and/or a microfibrous adhesive (not represented).

The absorbent material may be deposited as a single layer on one of the inner surface of one side of the core wrap. This is typically the case when the absorbent material is a mix of cellulose fibers and SAP particles. Alternatively, for airfelt-free cores, the absorbent layer 60 may be comprised of two absorbent sub-layers applied respectively on the substrate forming the top side 16 and bottom side 18 of the core, each of these layers being in a pattern of land areas separated by junction areas, as generally disclosed in WO2008/155699. These two absorbent layers when combined may form substantially continuous absorbent layer 60 of absorbent material. When the absorbent core is an airfelt-free absorbent core made according to such a process, a fibrous thermoplastic adhesive (not represented) is further used to immobilize the superabsorbent polymer particles on the substrates on which they are deposited.

### Channels and lateral absorbent zones

The absorbent layer 60 extends in a plane parallel to the transversal and longitudinal directions. The absorbent layer 60 comprises a first and a second longitudinally-extending channels 26a, 26b within its periphery. The channels are typically symmetrically disposed relative to the longitudinal centerline 80 of the core (disregarding any minor transversal shift due to usual process variation). The channels may be typically mirror image of each other relative to the longitudinal centerline.

The top side 16 of the core wrap is at least partially bonded to the bottom side 18 of the core wrap through the channels. For this purpose, the channels are typically substantially free of absorbent material so that channel bonds can be formed between the two sides of the core wrap. The channel bonds 27 are present within the channels. The channel bond 27 between the top side 16 and back side 18 of the core wrap may be provided by an auxiliary glue 72 applied directly to the inner surface of at least one of the top and/or bottom side of the core wrap, as illustrated in Fig. 2, and/or by any other bonding means such as mechanical bonds, fusion bonds, or ultrasonic bonds. Typically the channel bonds 27 may generally have the same outline and shape as the substantially absorbent material free channels 26 in which they are disposed. It is however not excluded that the channel bonds 27 may be provided in areas containing some absorbent material, in those cases the bonds may however be substantially less strong and more easily delaminate when the absorbent material swells.

As the absorbent material 60 swells when the core absorbs a liquid such as urine, the bond 27 in the channel 26 remain at least initially in place between the top and bottom sides of the core wrap, so that three-dimensional channels in the wet core are formed. This can cause the acquisition layer 52 above the absorbent core 28 to be deformed and form ditches corresponding to the underlying three-dimensional channels 26 at the surface of the topsheet 22. When the absorbent article comprises a distribution layer between the acquisition layer and the absorbent core, the distribution layer may also comprise channel areas free of distribution material and these may be at least partially superposed with the core channels (as illustrated for example by Roe et al. in WO2015/31225, WO2015/31229, WO2015/31243 or WO2015/31256).

The channels 26 extend substantially longitudinally, meaning that each channel extends more in the longitudinal direction (y) than in the transversal direction (x), and typically at least twice as much in the longitudinal direction than in the transverse direction (as measured after projection on the respective axis). The channels 26 may have a length L3 projected on the longitudinal centerline 80 that is at least 10% of the length L2 of the absorbent core, in particular from 20% to 80% of L2. The channels may have a width W3 along at least part of their length which is at least 2 mm, or at least 3 mm or at least 4 mm, up to for example 20 mm, or 16 mm or 12 mm. The width W3 of each channel may remain about constant through substantially the whole length of the channel, or it may vary along the length of the channel.

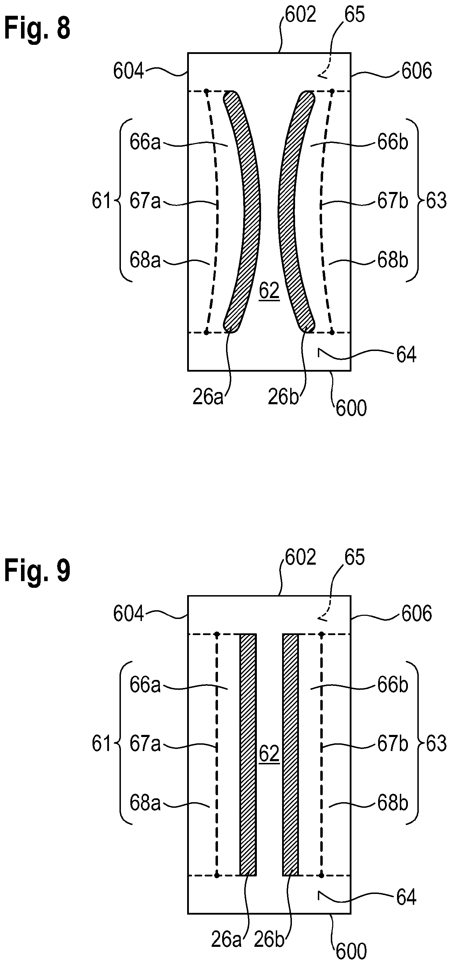

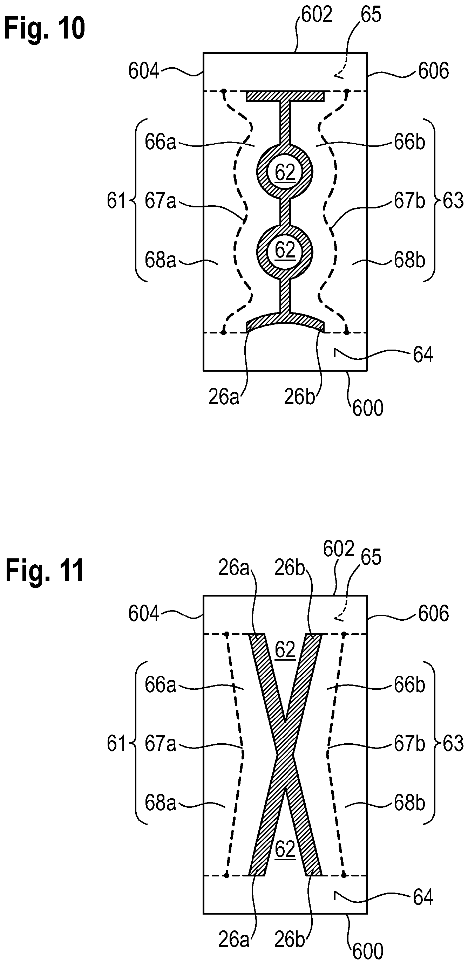

The channels may be at least partially curved. In particular, the channels may be concave towards the longitudinal centerline 80 in the crotch region of the article as illustrated in Fig. 4. The radius of curvature may typically be at least equal to the average transverse dimension of the absorbent material layer (and in particular at least 1.5 or at least 2.0 times this average transverse dimension). The radius of curvature may be constant or may vary along the length of the channel. The channels may alternatively be partially or entirely straight and parallel to the longitudinal direction (as illustrated in Fig. 9) or straight and under an angle of (e.g. from 5°) up to 30°, or for example up to 20°, or up to 10° with a line parallel to the longitudinal centerline. Also, and as illustrated in Figs. 10-13, the channels may comprise various shapes.

The channels 26a, 26b are typically symmetrically disposed relative to the longitudinal centerline. As illustrated in Figure 4, the first longitudinally-extending channel 26a and the second longitudinally-extending channel 26b may be distinct and entirely disposed in different side of the longitudinal centerline 80. The distance D between the two channels (measured transversally) may vary along their length or remain constant (as is typically the case when the channels are straight and parallel to the longitudinal direction as illustrated in Fig. 9). When the channels are entirely distinct from another, as in the examples of Figs. 8-9, the shortest spacing distance D between the channels may be for example at least 5 mm, or at least 10 mm, or at least 15 mm, or at least 20 mm, or up to 60 mm for example. It is however not excluded that the channels may be joined together, for example at their front or back extremities. In this case the largest distance D between the channels may be for example at least 10 mm, or at least 15 mm, or at least 20 mm, and up to 80 mm for example.

Furthermore, in order to reduce the risk of fluid leakages, the channels which are substantially free of absorbent material may advantageously not extend up to any of the edges of the absorbent material layer, and the channels are therefore surrounded by and fully encompassed within the absorbent material layer of the core. The smallest distance between a channel and the closest edge of the absorbent material layer may thus be at least 5 mm. Other executions are however possible as will be discussed later for the alternative executions.

The absorbent layer 60 is delimited by a periphery comprising a front edge 600, a back edge 602, and two longitudinal side edges 604, 606. As illustrated in Figures 8-13, the longitudinal side edges 604, 608 may be straight and parallel to the longitudinal direction. This was found to enable a more easily profiling of the absorbent material in the transversal direction to achieve a higher basis weight of absorbent material towards the respective channel than towards the respective longitudinal side edge wherein the first longitudinal side edge and the second longitudinal side edge. This may be particularly relevant for airfelt cores comprising a mixture of cellulose fibers and SAP particles as absorbent material, as a cellulose fiber containing material is not typically immobilized by a microfibrous glue, contrary to airfelt-free cores. While the absorbent layer may alternatively be shaped with a tapered section in the crotch region of the article (so-called shaped cores), this shaping reduces the width of the lateral absorbent zones and thus the possibility to efficiently profile the absorbent material distribution in the lateral absorbent zones.

The absorbent core 28 comprises a first lateral absorbent zone 61 and a second lateral absorbent zone 63, the lateral zone extending respectively transversally from the first channel 26a to the first longitudinal edge 604 of the absorbent layer, and from the second channel 26b to the second longitudinal edge 606 of the absorbent core. The absorbent core may advantageously comprise a central absorbent zone 62 disposed between the first channel 26a and the second 26b, as illustrated in Fig. 4 for example.

The central, first and second lateral absorbent zones comprise absorbent material. As defined herein, the central absorbent zone 62 and the lateral absorbent zones 61, 63 do not longitudinally extend beyond the longitudinal extremities of the channels 26a,b, and thus the central absorbent zone 62 and the lateral absorbent zones 61, 63 typically all have the same length L3, which is the length of the channels 26 (all length are measured parallel to the longitudinal direction unless indicated otherwise). The absorbent core may further comprise absorbent material in a front absorbent zone 64 extending longitudinally forward of the front extremities of the channels and up to the front end 600 of the absorbent layer and a back absorbent zone 65 extending longitudinally backward from the back extremities of the channels to the back end 602 of the absorbent layer.

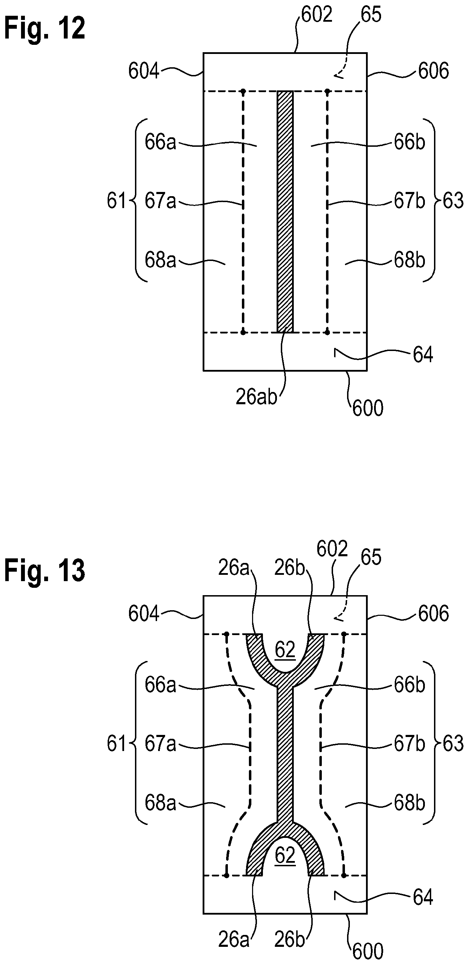

While the present invention is applicable to a variety of absorbent core and channel shape, the combined amount of absorbent material in the first and second lateral absorbent zones 61, 63 may typically range of from about 10% to 70% by weight of the total absorbent material in the absorbent core. The absorbent article may typically comprise a central absorbent zone 62 disposed between the first channel 26a and the second channel 26b. The amount of absorbent material in the central absorbent zone 62 may for example be in the range of from about 5% to about 30% of the total amount of absorbent material in the absorbent core. The front absorbent zone may comprise from about 0% to about 25% of the absorbent material, and the back absorbent zone from about 0% to about 25% of the absorbent material. The central absorbent zone 62 may in some channel design comprise more than one area, as illustrated for example in Figs. 10, 11, 13, or if the channels 26s are formed by a single straight channel (as illustrated in Fig. 12) there may be no central absorbent zone.

More generally, the amount of absorbent material may be for example distributed as indicated in Table 1 below, with particular ranges indicated in the last column. The percentage in Table 1 are reported by total weight of the absorbent material in the absorbent core.

**Table 1**

| | Typical range in weight % | In particular, in weight % |
|---|---|---|
| Front absorbent zone 64 | 0* - 25 | 5 - 20 |
| Central absorbent zone 62 | 5 - 30 | 10 - 20 |
| Lateral absorbent zone 61, 63 (combined) | 10 - 70 | 20 - 40 |
| Back absorbent zone 65 | 0* - 25 | 5 - 15 |

| | | |
|---|---|---|
| * although not preferred, it is possible that the channels extend up to the front and back edges of the absorbent core, so that the front and/or the back absorbent zones are not existent. | | |

Of course, the lengths of the different zones, the total amount of absorbent material and the basis weight distribution will be adapted for the intended usages of the absorbent articles considered. Keeping for example's sake the same general absorbent material distribution as shown in Fig. 4, the different absorbent zones may have the following, non-limiting, ranges of lengths and minimum and maximum average basis weight, with the lowest values adapted for smaller sizes of diapers and the larger values adapted for larger sizes of absorbent diapers.

**Table 2**

| | Length (in mm) | Average basis weight (g/m²) |
|---|---|---|
| Front absorbent zone 64 | 42-62 | 200-400 |
| Central absorbent zone 62 | 145-295 | 300 - 700 |
| Lateral absorbent zone 61, 63 | Same as central absorbent zone | 300-700 |
| Back absorbent zone 65 | 81-134 | 50-250 |

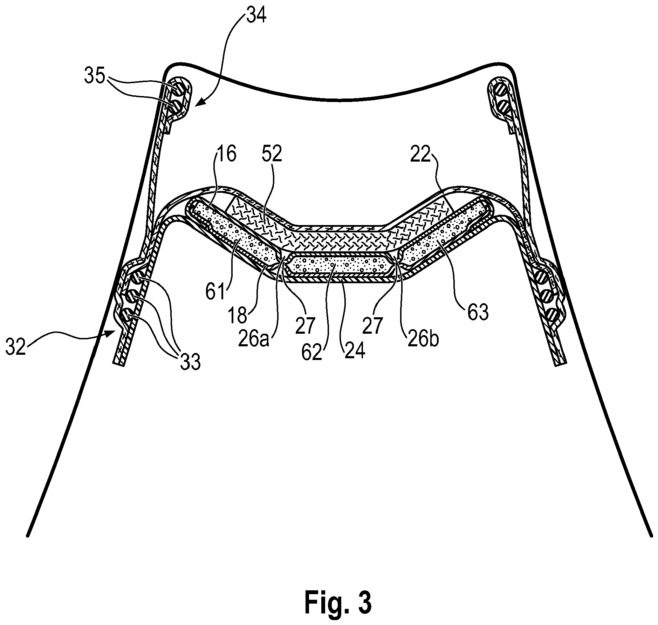

While Fig. 2 shows an exploded view where the channel bonds 27 are not represented, Fig. 3 shows an illustration of the curved shape that the chassis of the diaper takes when worn in the crotch region of an individual. The channel bonds may restrict to a certain extent the swelling of the absorbent material causing the chambers formed between the channels and/or between the channels and the longitudinal sides of the absorbent core to display some rigidity in the longitudinal direction while being flexible in the transversal direction. This effect is known in the art to reduce diaper sagging. Sagging is not desired as it can cause the barrier cuffs to lose contact with the skin and increase the risk of fluid leakage. According to the invention, it was found by the present inventors that even when sagging is reduced, the absorbent core can still assume a certain basis shape, where urine can pool at the bottom of the basin. Accordingly, the present inventors have found that it is advantageous to redistribute the absorbent material in the lateral absorbent zones of the absorbent core so that the first and second lateral absorbent zones are profiled in the transversal direction, in particular so that the basis weight of the absorbent material is higher towards the channel than towards the respective longitudinal side edge, as will be discussed below.

### Profiling in lateral absorbent zones

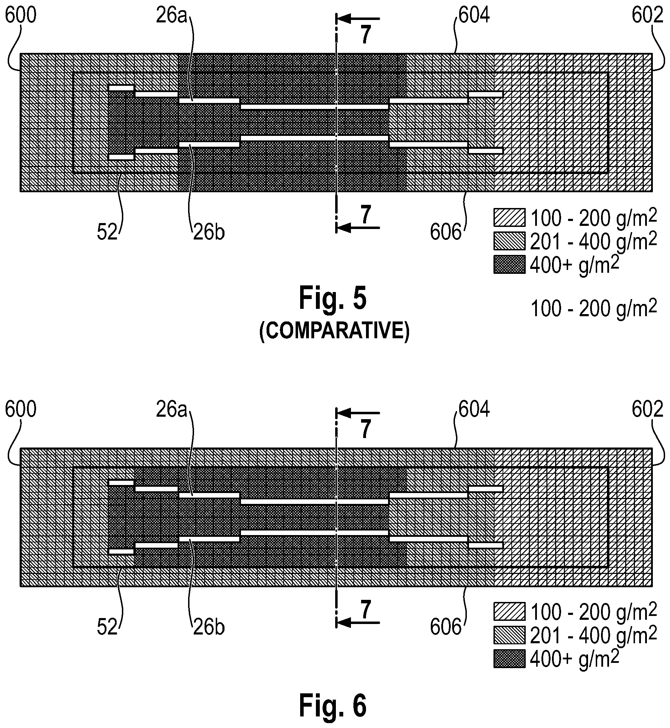

After having disclosed the general construction of an exemplary absorbent article and absorbent core in Figs.1-4, the improved distribution of absorbent material in the absorbent cores of the invention will be further generally described below, and by way of non-limiting illustrations with the embodiment shown in the Figures 5-7, with further alternative embodiments described in Figs. 8-13. Unless indicated otherwise, the features of the examples are not limiting the scope of the invention.

Fig. 5 schematically shows the distribution of absorbent material (SAP) in an exemplary comparative absorbent core. The local basis weight of the SAP material is illustrated using different shading according to different ranges, as indicated in the Figure. In this comparative core, the basis weight of the absorbent material in the lateral absorbent zones is generally constant in the transversal direction.

Fig. 6 shows schematically the distribution of absorbent material (SAP) in an exemplary absorbent core of the invention. As can be seen in this Fig. 6, in this inventive absorbent core, the basis weight of the absorbent material is substantially reduced in the regions of the absorbent lateral zone adjacent the longitudinal side edges 604,606, and the basis weight is substantially increased in the regions of the absorbent lateral zone adjacent the longitudinal channels.

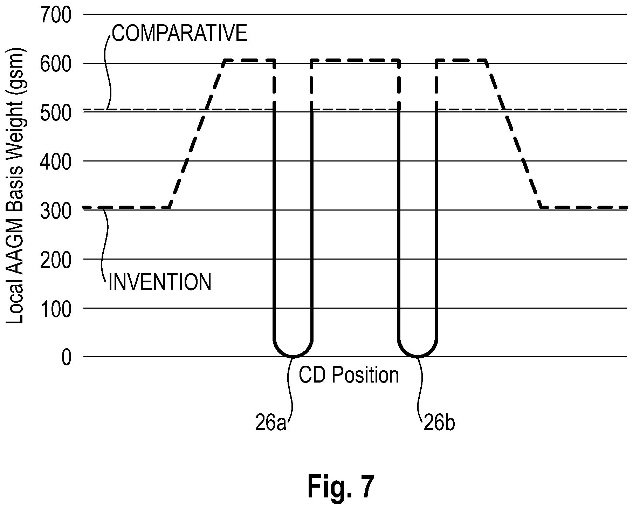

The difference in the transversal profiling of the absorbent material between the comparative core and the inventive core is further illustrated in Fig. 7, which shows the cross-sectional profiling of the absorbent material in the middle of the absorbent core.

As can be seen, also with reference to Fig. 7, the absorbent material in the comparative core is not profiled in the transversal direction in the lateral absorbent zones, whereas the inventive core shows a marked profiling in the lateral absorbent zones. The basis weight of the absorbent material in each lateral absorbent zone of the inventive core is higher towards the respective channel than towards the respective longitudinal side edge, at least along a substantial portion of the length of the channel (e.g. at least 50% of the length).

The profiling of the absorbent material in the lateral absorbent zones may be quantified using the Lateral Absorbent Zone Profile Ratio Test described further below. The Lateral Absorbent Zone Profile Ratio is preferably at least 1.40, as measured with the Lateral Absorbent Zone Ratio Test described herein, in particular from 1.40 to 3.0, for each of the first and second lateral absorbent zones.

Figs. 5-7 are to be understood as useful illustrations of how the basis weight of the absorbent material may be varied in the lateral absorbent zones of the absorbent core to achieve an absorbent material distribution of the present invention. However these should not be considered in any way limiting the scope of the invention, as other arrangements may be used to make the absorbent core of the invention.

By providing a relatively higher basis weight of absorbent material in the inner regions 66a, 66b of the lateral absorbent zones 61, 63 adjacent the channel 26a,b, the absorbent core can more efficiently absorbs urine as it was found that urine tends to pool in the transversal middle of the absorbent core when the article is worn. This is illustrated in Fig. 3. The absorbent core was found to significantly cave downwards when used. It was found that more absorbent capacity is required in the central absorbent zone and the inner region of the lateral absorbent zone disposed adjacent the channels than toward the longitudinal side edges of the lateral absorbent zones.

### Alternative channel shape

As illustrated in Figs. 1-8, the channels may be inwardly curved towards the longitudinal centerline 80. Alternatively, the channels may also be partially or entirely straight, and in particular longitudinally oriented parallel to the longitudinal centerline 80, as for example illustrated in Fig. 9. The profiled distribution of absorbent material according to the invention may also be useful when the channels 26 are straight and parallel to the longitudinal centerline as the curving of the absorbent core and pooling of urine at the bottom of the curve is also experienced for straight channels. Thus, this invention may also be useful when the channels are partially or entirely straight. This can provide a more flexible crotch portion of the absorbent core. Figures 8-9 are only exemplary, as other channel shapes are possible within the scope of the invention.

It is for example also not excluded that the curved channels instead of concavely curved as in two brackets towards each other ) (may also be convexly curved, as in two brackets facing away from each other ( ), so that the central absorbent zone is wider in the middle of the channels than at their front and back extremities. The channels may also be joined at one extremity (U shape), at both extremities (O shape) or form an X shape (see Fig. 11). It is also not excluded that the channels may comprise or consist of a straight channel disposed on the longitudinal centerline. The channels 26a,26b may consist in a single channel 26 as illustrated in Fig. 12. The channels may also comprise a portion that is straight and disposed on the longitudinal centerline and another portion that is curved (as illustrated for example on Figs. 10, 13).

### Core wrap 16, 18

The absorbent layer 60 is typically sandwiched between the top side 16 and the bottom side 18 of a core wrap. The absorbent layer is typically deposited on one or both inner sides of the core wrap during manufacture of the core, as is known in the art. Various core wrap constructions are possible. The core wrap may comprise as represented in the Figures two separate substrates forming substantially the top side 16 and the bottom side 18 of the core wrap respectively. Having two different substrates for example allows to deposit about half of the absorbent material on each substrate separately before combining these to form the core wrap, as is known from certain process for airfelt-free cores. The two substrates may be attached in a C-wrap configuration with two longitudinal seals 74, and optionally a front seal 280' and a back seal 282'. However this core wrap construction is not limiting of the invention, as any conventional core wrap construction may also be used, for example a single substrate on a portion of which the absorbent material is deposited and then the rest of the substrate folded over the deposited absorbent material to form the other side of the core. This single substrate construction can then be sealed longitudinally with a single longitudinal edge seal. The core wrap may also comprise two substrates disposed flat in a face-to-face relation (sandwich).

The core wrap may comprise any material suitable for receiving and containing the absorbent material. Typical substrate materials used in the production of conventional cores may be used, in particular paper, tissues, films, wovens or nonwovens, or laminate of any of these. The core wrap may be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 g/m2 to 15 g/m2. Suitable materials are for example disclosed in US7,744,576, US2011/0268932A1, US2011/0319848A1 and US2011/0250413A1. Nonwoven materials are typically made of synthetic fibers, such as PE, PET and in particular PP fibers. It is also possible that the core wrap may be at least partially formed from a component of the article having another function. For example, it is possible that the backsheet may form the bottom side of the core wrap and/or that a distribution layer or the topsheet may form the top side of the core wrap. However, typically the core wrap is made of one or more substrates whose only function is to receive and enclose the absorbent material, as indicated previously.

As used herein, the terms "nonwoven layer" or "nonwoven web" generally means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

As illustrated in Figure 2, a first substrate (the same reference number 16 is used as for the top side of the core wrap) may substantially form the whole of the top surface of the core wrap and a second substrate (same reference 18) substantially form the whole of the bottom surface of the core wrap, but it is not excluded that this may be the other way round. By "substantially forming the whole of the surface", it is meant that the outwardly extending flaps of the other substrate that have been folded longitudinally may also form part of the surface considered. The substrates are typically substantially planar in the same plane as the absorbent core, and each comprises an external surface and an internal surface. The internal surface is orientated towards the absorbent material and the external surface is the opposite surface. At least one of the substrate comprises at least one, and advantageously two outwardly extending flaps, which are folded around the front, back or side edges of the absorbent core and then attached to the external surface of the other substrate to form at least one so-called C-wrap seal. As seen in Fig. 2, the top substrate may comprise two side flaps laterally extending along the length of the core and which are folded inwardly over each side edge 284, 286 of the absorbent core. The flaps may be attached to the outer surface of the bottom side 18, for example by using an adhesive seal along each C-wrap seal 284', 286'. One or two continuous or semi-continuous lines of glue may be typically applied along the length of the flaps to bond the inner surface of the flaps to the external surface of the other substrate.

The absorbent core may also comprise so-called sandwich seals 280', 282' where the top side and bottom sides are bonded along one edge of the core to each other in face-to-face relationship with the inner surface of one side bonded to the inner surface of the other side. These sandwich seals can for example be formed using a hotmelt glue applied in a series of stripes in a direction perpendicular of the edge, as shown on the front edge 280 and back edge 282 of the core on Fig. 4 for example.

The substrates may typically be commercially supplied as rolls of material of several hundred meters of length. Each roll is then integrated in the converting line and unrolled at high speed while the auxiliary adhesive, the absorbent material and the fibrous thermoplastic adhesive layer if present are deposited or applied on the substrate and then further converted into an absorbent core when a core wrap enclosing the absorbent material is formed by the second substrate. Typically the machine direction (MD) of the converting line may correspond to the longitudinal direction (y) of the substrate/core and the cross-machine direction (CD) to the transversal direction (x) of the substrate/core. The substrates may be cut along the front and back edges of the core 280, 282 to individualize the core.

### Absorbent material

The absorbent material may be any known absorbent material known in the art, but will typically comprise or consist of superabsorbent polymers (herein referred to as "SAP"). The SAP may be typically in particulate forms (superabsorbent polymer particles), optionally mixed with cellulose fibers, but it is not excluded that other forms of SAP may be used such as a superabsorbent polymer foam for example. The SAP useful in the present invention includes a variety of water-insoluble, but water-swellable polymers capable of absorbing large quantities of fluids.

The term "superabsorbent polymer" refers herein to absorbent materials, which may be cross-linked polymeric materials, that can typically absorb at least 10 times their weight of an aqueous 0.9% saline solution as measured using the Centrifuge Retention Capacity (CRC) test (EDANA method WSP 241.2.R3 (12). The SAP may in particular have a CRC value of more than 20 g/g, or more than 24 g/g, or of from 20 to 50 g/g, or from 20 to 40 g/g, or 24 to 30 g/g.

The absorbent material may comprise a relative high amount of SAP, in particular the absorbent material may comprise at least 80%, in particular at least 85%, 90%, 95% and up to 100% of SAP by weight of the absorbent material. The absorbent material may in particular be free or substantially free of cellulose fibers, such as less than 20%, in particular less than 10%, 5% or even 0% of cellulose fibers by weight of the absorbent material. The absorbent material may thus consist or consist essentially of SAP. The core wrap is not considered as absorbent material for the purpose of calculating the percentage of SAP in the absorbent core. When the absorbent material comprises cellulose fibers, the content of SAP may typically range from 60% to 80% by weight of the absorbent material.

The superabsorbent polymers may be in particulate form, so as to be flowable in the dry state and thus easily deposited on a substrate. Typical particulate absorbent polymer materials are made of poly(meth)acrylic acid polymers. However, starch-based particulate absorbent polymer materials may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/or surface cross-linked. Suitable materials are described in WO 07/047598, WO 07/046052, WO 2009/155265 and WO 2009/155264. Suitable superabsorbent polymer particles may be obtained by current state of the art production processes, for example as described in WO 2006/083584. The superabsorbent polymers are preferably internally cross-linked, i.e. the polymerization is carried out in the presence of compounds having two or more polymerizable groups which can be free-radically copolymerized into the polymer network. In some embodiments, the SAP are formed from polyacrylic acid polymers/ polyacrylate polymers, for example having a neutralization degree of from 60% to 90%, or about 75%, having for example sodium counter ions.

The SAP particles may be relatively small (under 1 mm in their longest dimension) in their dry state and may be roughly circular in shape, but granules, fibers, flakes, spheres, powders, platelets and other shapes and forms are also known to persons skilled in the art. Typically, the SAP particles may be in the form of spherical-like particles. In contrast to fibers, "spherical-like particles" have a longest and a smallest dimension with a particulate ratio of longest to smallest particle dimension in the range of 1-5, where a value of 1 would equate a perfectly spherical particle and 5 would allow for some deviation from such a spherical particle. The superabsorbent polymer particles may have a particle size of less than 850 µm, or from 50 µm to 850 µm, preferably from 100 µm to 710 µm, more preferably from 150 µm to 650 µm, as measured according to EDANA method WSP 220.2-05. SAP having a relatively low particle size help to increase the surface area of the absorbent material which is in contact with liquid exudates and therefore support fast absorption of liquid exudates.

The absorbent core will typically comprise only one type of SAP, but it is not excluded that a blend of different SAPs may be used. The fluid permeability of a superabsorbent polymer can be quantified using its Urine Permeability Measurement (UPM) value, as measured in the test disclosed in US2014/005622A1. The UPM of the SAP may for example be of at least 10 ×10-7 cm³.sec/g, or at least 30 ×10⁻⁷ cm³.sec/g, or at least 50 ×10-7 cm³.sec/g, or more, e.g. at least 80 or 100 ×10⁻⁷ cm³.sec/g. The SAP particles may have a time to reach an uptake of 20 g/g (T20) of less than 240 s, preferably from 40s to less than 240 s, more preferably from 65 s to 215 s, as measured according to the K(t) test method as described in WO2015/041784 (Peri et al).

The absorbent material may be deposited on any of the core wrap substrates using known techniques, which may allow relatively precise deposition of absorbent material at relatively high speed. In particular the SAP printing technology as disclosed for example in US2006/024433 (Blessing), US2008/0312617 and US2010/0051166A1 (both to Hundorf et al.) may be used. This technique uses a transfer device such as a printing roll to deposit SAP onto a substrate disposed on a grid of a support which may include a plurality of cross-bars extending substantially parallel to each other and spaced apart from one another. The channels 26, which are substantially free of absorbent material, and through which the bonding 27 is executed, can be formed for example by modifying the pattern of the grid and receiving drums so that no SAP particles is applied in the selected areas, as exemplary disclosed in US2012/0312491 (Jackels). This technology allows high-speed and precise deposition of SAP on a substrate in particular to provide one or more area(s) substantially free of absorbent material surrounded by absorbent material.

In many applications, the liquid discharge occurs predominantly in one area of the core. For diapers, the liquid may predominantly be released towards the crotch region of the core and to a lesser extent the front of the core. Relatively less liquid may be released towards the back of the core. Thus it may be beneficial to profile the amount of absorbent material along the longitudinal direction of the absorbent structure so that more absorbent material is present in the areas where the liquid is more likely to insult the core.

### Auxiliary glue 72

The auxiliary glue 72 is optional but is advantageous to immobilize the absorbent material 60 and/or at least partially contribute to form channel bonds 27. When present, the auxiliary glue 72 may be applied directly over the inner surface of one or both of the top side and bottom side of the core wrap. The auxiliary glue may at least partially form the bonds 27 between the top side and the bottom side of the core wrap.

The auxiliary glue may comprise or consist of any kind of thermoplastic hot-melt adhesives used in the field of absorbent core making. Such an adhesive generally includes one or more polymers to provide cohesive strength (e.g., aliphatic polyolefins such as ethylene-propylene copolymers, polyetheramides, polyetheresters, and combinations thereof; ethylene vinyl acetate copolymers; styrene-butadiene or styreneisoprene block copolymers; etc.), a resin or analogous material (sometimes called a tackifier) to provide adhesive strength (e.g., hydrocarbons distilled from petroleum distillates; rosins and/or rosin esters; terpenes derived, for example, from wood or citrus, etc.); and optional waxes, plasticizers or other materials to modify viscosity (e.g., mineral oil, polybutene, paraffin oils, ester oils, and the like), and/or other additives including, but not limited to, antioxidants or other stabilizers. Exemplary suitable commercial adhesives are available from Fuller under reference number 1358LO and from Henkel under reference numbers DM3800 and DM526. The auxiliary glue can be applied by any adhesive applicator known in the field, in particular bead, slot or spray nozzles.

When the absorbent material consists of SAP particles, it may be further immobilized by a microfibrous adhesive net which is sprayed on the SAP particles. Exemplary commercial adhesives which are suitable as auxiliary glue and/or microfibrous adhesive are NW1151 ex. HB Fuller and H2898 ex. Bostik.

The auxiliary glue may advantageously overlap with the channels, the central absorbent zone and at least a portion of each of the lateral absorbent zone which is adjacent the channels, that is the portion of the lateral absorbent zone where the absorbent material is in higher amount per unit of area than in the rest of the lateral absorbent zone. The auxiliary glue may also extend widthwise to substantially cover the whole width of the absorbent core (the auxiliary glue width may thus be in the range of from 50% to 100% of W2).

The auxiliary glue 72 was discussed above with reference to the top side 16 of the core wrap which is placed towards the topsheet 22 in the finished absorbent article 20. This is however not limiting, as the auxiliary glue may be disposed instead or additionally on the inner surface of the bottom side of the core wrap.

### Exemplary method and apparatus for making the absorbent core

The absorbent cores of the invention may be made by any conventional methods known in the art that allow a relative precise and controlled deposition of absorbent material. The articles may be hand-made or industrially produced at high speed on a modern converting line. As mentioned above, the absorbent core of the invention can in particular be made industrially by combining two absorbent structures and using the SAP printing method first disclosed in WO2008/155699 (Hundorf et al.) and further developed in WO2012/170798A1 (Jackels et al.), with the adaptations required to obtain the specific SAP distribution of the invention. The absorbent core comprising channels may be also made by deposition of a mixture of cellulose fibers and SAP particles onto a mold cavity having a profiled depth, as is known in the art for making absorbent core comprising a mixture of cellulose fibers and SAP particles, such as disclosed in EP3,342,386 (Ontex) or WO2007/122,526 (Kimberly-Clark) for example.

### Topsheet 22

The topsheet 22 typically forms the majority of the wearer-contacting surface of the article and is the first layer that the body exudates contact. The topsheet is preferably compliant, soft-feeling, and non-irritating to the wearer's skin. Further, at least a portion of the topsheet is liquid permeable, permitting liquids to readily penetrate through its thickness. Any known topsheet may be used in the present invention. A suitable topsheet may be manufactured from a wide range of materials. Most topsheet are nonwoven materials or apertured formed films, but other material are possible such as porous foams, reticulated foams, woven materials. Typical diaper topsheet have a basis weight of from about 10 g/m² to about 28 g/m², in particular between from about 12 g/m² to about 18 g/m² but higher basis weights are possible if it is desired to provide a very soft feeling wearer-contacting surface for example. The topsheet may also be treated with a wetting agent to make it more hydrophilic.

### Backsheet 24

The backsheet is typically the outermost layer of the article and acts as a barrier for body exudates contained in the absorbent article. The backsheet may be positioned directly adjacent the garment-facing surface of the absorbent core. The backsheet is typically impermeable, or at least substantially impermeable, to liquids (e.g., urine). The backsheet may, for example, be or comprise a thin plastic film such as a thermoplastic film having a thickness of about 0.012 mm to about 0.051 mm. The basis weight of those films is usually as low as possible to save material costs, typically from 10 gsm to 30 gsm, in particular below 20 gsm. A covering low basis weight nonwoven may be attached to the external surface of the film to provide for a softer touch.

Suitable backsheet materials include breathable materials which permit vapors to escape from the absorbent article while still preventing, or at least inhibiting, exudates from passing through the backsheet. Example breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, VA, and sold under the designation EXAIRE, and monolithic films such as manufactured by Clopay Corporation, Cincinnati, OH under the name HYTREL blend P18-3097.

### Acquisition layer 52

The absorbent article 20 may comprise an acquisition layer, whose function is to quickly acquire the fluid away from the topsheet so as to provide a good dryness for the wearer. The acquisition layer is typically placed directly under the topsheet. Optionally, a second acquisition layer, typically referred to as distribution layer, may be at least partially disposed under the acquisition layer. The acquisition layer may typically be or comprise a non-woven material, for example a SMS or SMMS material, comprising a spunbonded, a melt-blown and a further spunbonded layer, or a spunlaced nonwoven, or alternatively a carded chemical-bonded nonwoven. The non-woven material may in particular be latex bonded. Exemplary upper acquisition layers are disclosed in US7,786,341. Carded, resin-bonded nonwovens may be used, in particular where the fibers used are solid round or round and hollow PET staple fibers (50/50 or 40/60 mix of 6 denier and 9 denier fibers). An exemplary binder is a butadiene/styrene latex. Nonwovens have the advantage that they can be manufactured outside the converting line and stored and used as a roll of material. Further useful nonwovens are described in US6,645,569 (Cramer et al.), US6,863,933 (Cramer et al.), US7,112,621 (Rohrbaugh et al.), US2003/148684 (Cramer et al.) and US2005/008839 (Cramer et al.). The acquisition layer may be stabilized by a latex binder, for example a styrene-butadiene latex binder (SB latex). Processes for obtaining such latices are known, for example, from EP 149880 (Kwok) and US 2003/0105190 (Diehl et al.). The binder may typically be present in the acquisition layer in amount ranging from about 12% to about 50%, for example about 30%, by total weight of the acquisition layer. SB latex is available under the trade name GENFLO^{™} 3160 (OMNOVA Solutions Inc.; Akron, Ohio).

Another typical acquisition layer, sometimes referred to as secondary topsheet, may for example be a through-air bonded carded web ("TABCW") but many other alternatives material are known in the art and may be used instead. "Bonded carded web" refers to webs that are made from staple fibers that are sent through a combing or carding unit, which breaks apart and aligns the staple fibers in the machine direction to form a generally machine direction-oriented fibrous nonwoven web. This web is then drawn through a heated drum, creating bonds throughout the fabric without applying specific pressure (thru air bonding process). The TABCW material provides a low density, lofty through-air bonded carded web. The web may for example have a specific weight basis level at about 15 g/m² to about 120 g/m², in particular about 30 g/m² to about 80 g/m². The TABCW material can for example comprise about 3 to about 10 denier staple fibers. Examples of such TABCW are disclosed in WO2000/71067 (KIM DOO-HONG et al.). TABCW are available directly from all usual suppliers of nonwoven webs for use in absorbent articles, for example Fitesa Ltd or Fiberweb Technical Nonwovens.

The acquisition layer 52 of the invention advantageously overlaps with all, or at least 50% of the absorbent layer 60 of the absorbent core. The acquisition layer also preferably extends transversally to overlap with a portion of the lateral absorbent zones, in particular a significant proportion of the region of the lateral absorbent zone adjacent to the channel. Advantageously, the acquisition layer does not extend beyond or even up to the longitudinal edges 604, 606 of the absorbent layer 60. Fig. 6 for example shows the boundaries of the acquisition layer 72, that is superposed with the inner region of the lateral absorbent zones that comprises the higher basis weight distribution of absorbent material.

### Distribution layer

The absorbent article may also comprise a distribution layer, typically in addition to an acquisition layer, whose function is to spread the insulting fluid liquid over a larger surface within the article so that the absorbent capacity of the core can be more efficiently used, especially when the absorbent core is an airfelt-free. The distribution layer may for example comprise at least 50% by weight of cross-linked cellulose fibers. The cross-linked cellulosic fibers may be crimped, twisted, or curled, or a combination thereof including crimped, twisted, and curled. This type of material has been used in the past in disposable diapers as part of an acquisition system, for example US 2008/0312622 A1 (Hundorf). The cross-linked cellulosic fibers provide higher resilience and therefore higher resistance against the compression in the product packaging or in use conditions, e.g. under baby weight. The distribution layer may itself comprise material-free channels at least partially superposed with the absorbent core channels.

Exemplary chemically cross-linked cellulosic fibers suitable for a distribution layer are disclosed in US5,549,791, US5,137,537, WO95/34329 or US2007/118087. Exemplary cross-linking agents include polycarboxylic acids such as citric acid and/or polyacrylic acids such as acrylic acid and maleic acid copolymers.

The distribution layer comprising cross-linked cellulose fibers may comprise other fibers, but this layer may advantageously comprise at least 50%, or 60%, or 70%, or 80%, or 90% or even up to 100%, by weight of the layer, of cross-linked cellulose fibers (including the cross-linking agents).

### Fastening system 42, 44

The absorbent article may include a fastening system, especially when the article is a taped diaper as exemplified in Fig. 1. The fastening system can be used to provide lateral tensions about the circumference of the absorbent article to hold the absorbent article on the wearer. Such a fastening system is not necessary for absorbent pant articles since the waist region of these articles is already bonded and elasticized. The fastening system usually comprises a fastener 42 such as tape tabs, hook and loop fastening components, interlocking fasteners such as tabs & slots, buckles, buttons, snaps, and/or hermaphroditic fastening components, although any other known fastening means are generally acceptable. A landing zone 44 is normally provided on the front waist region of the article for the fastener 42 to be releasably attached. Some exemplary surface fastening systems are disclosed in US3,848,594, US4,662,875, US4,846,815, US4,894,060, US4,946,527, US5,151,092 and US5,221,274 (Buell). An exemplary interlocking fastening system is disclosed in US 6,432,098. The fastening system may also provide a means for holding the article in a disposal configuration as disclosed in US4,963,140 (Robertson et al.)

The fastening system may also include primary and secondary fastening systems, as disclosed in US4,699,622 to reduce shifting of overlapped portions or to improve fit as disclosed in US5,242,436, US5,499,978, US5,507,86, and US5,591,152.

### Front and back ears 46, 40

The absorbent article may comprise front ears 46 and back ears 40, as is known in the art in taped diapers. Training pants which are already sealed along the waist edges typically do not require front ears and back ears. The ears can be integral part of the chassis, for example formed from the topsheet and/or backsheet as side panel. Alternatively, as represented in Fig. 1, they may be separate elements attached by gluing and / or heat embossing to the chassis of the diaper. The back ears 40 are optionally stretchable to facilitate the attachment of the tabs 42 on the landing zone 44 and maintain the taped diapers in place around the wearer's waist. The front ears 46 may also be optionally elastic or extensible to provide a more comfortable and contouring fit.

### Barrier leg cuffs 34 and gasketing cuffs 32

Absorbent articles such as taped diapers, training pants or adult incontinence pants may typically further comprise cuff components 30 that improve the fit of the article around the legs of the wearer, in particular the cuffs typically comprise barrier leg cuffs 34 and gasketing cuffs 32. The cuffs 30 may comprise a piece of material, typically a nonwoven, which is one side partially bonded to the article and on the other side can be partially raised away from the topsheet and thus stand up from the plane defined by the topsheet as shown for example in Fig. 2. Both parts of the cuffs may be advantageously elasticized. The raised part of the cuff components is referred to herein as barrier leg cuffs 34 and can provide improved containment of liquids and other body exudates approximately at the junction of the torso and legs of the wearer. The barrier leg cuffs 34 extend at least partially between the front edge and the back edge of the absorbent article on opposite sides of the longitudinal centerline and are at least present adjacent to the crotch point (C).

For example, US3,860,003 describes a disposable diaper which provides a contractible leg opening having a side flap and one or more elastic members to provide an elasticized leg cuff (a gasketing cuff). US4,808,178 (Aziz) and US4,909,803 (Aziz) describe disposable diapers having "stand-up" elasticized flaps (barrier leg cuffs) which improve the containment of the leg regions. US4,695,278 (Lawson) and US4,795,454 (Dragoo) describe disposable diapers having dual cuffs, including gasketing cuffs and barrier leg cuffs. All or a portion of the barrier leg and/or gasketing cuffs may be treated with a lotion.

The barrier leg cuffs 34 may be delimited by a proximal edge 36 joined to the rest of the article, typically the topsheet, and a free terminal edge 38 intended to contact and form a seal with the wearer's skin. The barrier leg cuffs 34 may be joined at the proximal edge 36 with the chassis of the article by a bond 37 which may be made for example by adhesive bonding, fusion bonding or combination of known bonding means, for example as disclosed in WO2014/168810A1 (Bianchi et al.). The bond 37 at the proximal edge 36 may be continuous or intermittent.

The barrier leg cuffs 34 can be integral with (i.e. formed from) the topsheet or the backsheet, or more typically be formed from a separate material 30 joined to the rest of the article. Typically the material of the barrier leg cuffs may extend through the whole length of the article but is "tack bonded" to the topsheet towards the front edge and back edge of the article so that in these sections the barrier leg cuff material remains flush with the topsheet. Each barrier leg cuff 34 may comprise one, two or more elastic strings 35 close to its free terminal edge 38 to provide a better seal.

In addition to the barrier leg cuffs 34, the article may comprise gasketing cuffs 32, which are formed in the same plane as the chassis of the absorbent article, in particular may be at least partially enclosed between the topsheet and the backsheet, and typically placed further laterally outwardly relative to the barrier leg cuffs 34. The gasketing cuffs 32 can provide a better seal around the thighs of the wearer. Usually each gasketing leg cuff 32 will comprise one or more elastic string or elastic element 33 comprised in the chassis of the diaper for example between the topsheet and backsheet in the area of the leg openings. Typically the barrier leg cuffs 34 are disposed more internally than the gasketing cuffs 32. The barrier leg cuffs are thus also referred to as inner cuffs and the gasketing cuffs as outer cuffs.

### Other components

The absorbent articles of the invention can further comprise any other typical components known for the intended purpose of the article that are not illustrated in the Figures, such as a transverse barrier element extending across the topsheet to form a receptacle for bowel movement, a lotion application on the topsheet, a wetness indicator comprising a pH indicator disposed between the absorbent core and the backsheet, etc. These components are well-known in the art and will not be further discussed herein. Reference is made to WO2014/093310 where several examples of these components are disclosed in more details.

The absorbent article may also comprise at least one elastic waistband or elastic waist cuff (referred together as elastic waist feature) disposed parallel to and along the back edge of the article and/or also parallel to and along the front edge of the article. Such elastic waist features help providing improved fit and containment at the back and/or front edge of the article. The elastic waist feature is generally intended to elastically expand and contract to dynamically fit the wearer's waist. The elastic waist feature may be constructed in different configurations. Non-limiting examples of back and front waistbands can be found in WO2012/177400 and WO2012/177401 (Lawson), and US4,515,595, US4,710,189, US5,221,274 and US6,336,922 (VanGompel et al.).

### Packages

A plurality of articles according to the invention may be packaged in a package for transport and sale. At least 50% of the articles in the package may be according to the invention, and preferably substantially all the articles. The articles may be folded and packaged as is known in the art. The package may be for example a plastic bag or a cardboard box. Diapers may typically bi-folded along the transversal centerline and the ears folded inwardly before being packaged. The absorbent articles may be packed under compression so as to reduce the size of the packages, while still providing an adequate number of absorbent articles per package. By packaging the absorbent articles under compression, caregivers can easily handle and store the packages, while also providing distribution and inventory savings to manufacturers owing to the size of the packages.

The absorbent articles may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression may be typically sampled from the production line between the folding unit and the stack packing unit. The stack height before compression is measured by taking 10 articles before compression and packing, and measuring their stack height as indicated for the IBSH.

Packages of the absorbent articles of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby, according to the In-Bag Stack Height Test described herein. For each of the values indicated in the previous sentence, it may be desirable to have an In-Bag Stack Height of greater than 60, or greater than 70 mm, or greater than 75 mm, or greater than 80 mm. Alternatively, packages of the absorbent articles of the present disclosure may have an In-Bag Stack Height of from 60 mm to 110 mm, from 75 mm to 110 mm, from 80 mm to 110 mm, from 80 mm to 105 mm, or from 80 mm to 100 mm, specifically reciting all 0.1 mm increments within the specified ranges and all ranges formed therein or thereby, according to the In-Back Stack Height Test described herein.

### Relations between the layers and components

Typically, adjacent layers will be joined together using conventional bonding method such as adhesive coating via slot coating or spraying on the whole or part of the surface of the layer, or thermo-bonding, or pressure bonding or combinations thereof. Most of the bonding between components is for clarity and readability not represented in the Figure. Bonding between the layers of the article should be considered present unless specifically excluded. Adhesives may be typically used, for example between the backsheet and the core wrap. The adhesives used may be any standard hotmelt glue as known in the art. The individual components may be converted into an absorbent article according to any process as is known in the art.

### Examples and data

### Performance data

Different sets of absorbent cores were made on a production line. Some absorbent cores, referred to as Comparative, had non-transversally profiled lateral absorbent zones, as illustrated in Fig. 5, while other absorbent cores, referred to as Inventive, had transversally profiled lateral absorbent zones as illustrated in Fig. 6. The amount of SAP was also differentiated, as indicated in Table 3. The different absorbent cores were otherwise constructed similarly, with the absorbent material consisting of the same type of SAP particles with two similarly bonded longitudinal channels. These airfelt-free cores were made using the SAP-printing process referred above (WO2012/170778A1, Rosati et al.). The absorbent layer 60 in both sets was generally rectangular as illustrated in the Figures 5-6. The absorbent cores were integrated in a Size 4 taped diaper chassis on the production line corresponding to commercially marketed Pampers^{®} Baby-Dry^{®} in Europe. The construction of the taped diapers produced were generally similar to the taped diaper illustrated in Figs. 1-2.

The diapers were tested with panelists under obligations of confidentiality having babies in the age of 9 months to 24 months. The panelists were asked to record events of urine leakage during the day as well as during the night, and specifically if the leakage was at the front of the diaper. The results are summarized in Table 3 below, where the amount of SAP used for each diaper is further indicated.

**Table 3**

| | Comparative 1 | Inventive 1 | Comparative 2 | Inventive 2 |
|---|---|---|---|---|
| SAP amount | 13.0 g | 12.0 g | 12.4 g | 12.4 g |
| Urine Leakage % | 3.6% | 3.4% | 4.7% | 3.6% |
| Front Leakage % | 2.0% | 1.6% | 2.5% | 1.6% |
| Night Leakage | 7.8% | 6.9% | 10.0% | 7.6% |
| Front Night Leakage | 4.1% | 3.2% | 6.1% | 3.2% |

Comparing the leakage data of Comparative 1 and Inventive 1, it can be seen that even with a 1.0 g reduced amount of SAP, the inventive diaper was not worse than the comparative diaper, and even had slightly reduced urine leakage. Comparing the data obtained between Comparative 2 and Inventive 2, it can be seen that the inventive diaper had significantly reduced occurrence of front leakage despite this example being at parity SAP level with the comparative core.

### Lateral Absorbent Zone Profile Ratio Data

The Lateral Absorbent Zone Profile Ratio of various absorbent cores was measured according to the Lateral Absorbent Zone Profile Ratio Test described below. In short, this test provides one way to measure the transversal profiling of a lateral absorbent zone by comparing the weight of the inner half of the lateral absorbent zone, which is closest to the channel, to the weight of the outer half of the lateral absorbent zone, which is closest to the longitudinal edge of the absorbent layer.

Four commercial diaper products were measured according to the Test:
Commercial 1: DM^{®} Babylove^{®} Premium S4, Lot291223 SE071729
Commercial 2: DM^{®} Nature HBD3 07:43 2023.12.12 EXP 12/2016 015
Commercial 3: Hipp^{®} BabySanft, S2 K00882315:51
Commercial 4: Huggies^{®} S4 Israel Freedom Dry, EXP 13/10/2026 07:33

These commercial diaper products were airfelt diapers (the absorbent material is a mix of cellulose fibers and SAP) having a shaped core. Commercial 1, 2 and 4 had two parallel straight channels (similar to Fig. 9). The channels of Commercial 3 had a shape as illustrated in Fig. 13.

Additionally, two comparative examples corresponding to marketed Pampers taped and pant diapers having an airfelt-free cores with two curved channels were tested (these comparative products were made on the same experimental line as the Inventive Examples above, i.e. according to commercial specification).

The inventive example below is the same absorbent article as Inventive 2 in the Performance data section above, containing 12.4 g SAP. Left and Right lateral absorbent zones were separated and inner half and outer half weighted to calculate the Left Ratio (R Left) and the Right Ratio (R Right) according to the Lateral Absorbent Zone Profile Ratio Test described below.

**Table 4**

| Product | Wo Left (g) | Wi Left (g) | Wi Right (g) | Wo Right (g) | R Left (WiL / WoL) | R Right (WiR / WoR) |
|---|---|---|---|---|---|---|
| Commercial 1 | 0.89 | 1.02 | 1.45 | 0.99 | 1.15 | 1.48 |
| Commercial 2 | 0.99 | 1.11 | 1.00 | 0.73 | 1.13 | 1.38 |
| Commercial 3 | 2.04 | 2.16 | 2.07 | 1.63 | 1.06 | 1.27 |
| Commercial 4 | 0.57 | 0.74 | 0.62 | 0.39 | 1.30 | 1.58 |
| Comparative PAMPERS^{®} Size 4 Taped | 1.64 | 1.99 | 1.91 | 1.61 | 1.22 | 1.20 |
| Comparative PAMPERS^{®} Premium Pants S5 | 1.34 | 1.63 | 1.61 | 1.45 | 1.22 | 1.12 |
| Inventive 2 | 1.34 | 2.23 | 2.18 | 1.40 | 1.66 | 1.57 |

As can be seen from Table 4, the absorbent material distribution in commercial airfelt diapers can significantly differ between the left and right lateral absorbent zones. Applicant believes that this may be due to the left and right sides of the absorbent core being submitted to highly different acceleration factors in the folding or transfer process on the converting line. This can cause the absorbent material to be displaced from the original placement in the lateral absorbent zone in a non-uniform way between the left and right lateral absorbent zones, especially for shaped cores. Thus, while the absorbent material may be distributed uniformly in the transversal direction in the lateral absorbent zones when the core is formed, the absorbent material may be displaced in a non-controlled manner in the end product. Airfelt-free cores comprising SAP immobilized by a microfibrous adhesive had a less variable distribution between the two lateral absorbent zones.

The inventive example on the other hand had significantly more profiled absorbent material distribution in the first and second lateral absorbent zones as quantified by the Lateral Absorbent Zone Profile Ratio measurements. Only the inventive example had a Lateral Absorbent Zone Profile Ratio of at least 1.40 in each one of the first (left) and second (right) lateral absorbent zone.

### Test procedures

The values indicated herein are measured according to the methods indicated herein below, unless specified otherwise. All measurements are performed at 21°C ± 2°C and 50% ± 5% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### Centrifuge Retention Capacity (CRC)

The CRC measures the liquid absorbed by the superabsorbent polymer particles for free swelling in excess liquid. The CRC is measured according to EDANA method WSP 241.2.R3 (12).

### Dry Absorbent Core Caliper Test

This test may be used to measure the caliper of the absorbent core (before use i.e. without fluid loading) in a standardized manner.

Equipment: Mitutoyo manual caliper gauge with a resolution of 0.01 mm, or equivalent instrument.

Contact Foot: Flat circular foot with a diameter of 17.0 mm (± 0.2 mm). A circular weight may be applied to the foot (e.g., a weight with a slot to facilitate application around the instrument shaft) to achieve the target weight. The total weight of foot and added weight (including shaft) is selected to provide 2.07 kPa (0.30 psi) of pressure to the sample.

The caliper gauge is mounted with the lower surface of the contact foot in an horizontal plane so that the lower surface of the contact foot contacts the center of the flat horizontal upper surface of a base plate approximately 20 × 25 cm. The gauge is set to read zero with the contact foot resting on the base plate.
Ruler: Calibrated metal ruler graduated in mm.
Stopwatch: Accuracy 1 second.

Sample preparation: The core is conditioned at least 24 hours as indicated above.

Measurement procedure: The core is laid flat with the bottom side, i.e. the side intended to be placed towards the backsheet in the finished article facing down. The point of measurement (e.g. the crotch point C) is carefully drawn on the top side of the core taking care not to compress or deform the core.

The contact foot of the caliper gauge is raised and the core is placed flat on the base plate of the caliper gauge with the top side of the core up so that when lowered, the center of the foot is on the marked measuring point.

The foot is gently lowered onto the article and released (ensure calibration to "0" prior to the start of the measurement). The caliper value is read to the nearest 0.01 mm, 10 ± 1 seconds after the foot is released.

The procedure is repeated for each measuring point. If there is a fold at the measuring point, the measurement is done in the closest area to this point but without any folds. Ten articles are measured in this manner for a given product and the average caliper is calculated and reported with an accuracy of one tenth mm.

### Absorbent Article Caliper Test

The Absorbent Article Caliper Test can be performed as for the Dry Absorbent Core Caliper Test with the difference that the caliper of the finished absorbent article is measured instead of the caliper of the core. The point of measurement may be the intersection of the longitudinal centerline 80 and transversal centerline 90 of the absorbent article. If the absorbent articles were provided folded and/or in a package, the articles to be measured are unfolded and/or removed from the center area of the package. If the package contains more than 4 articles, the outer most two articles on each side of the package are not used in the testing. If the package contains more than 4 but fewer than 14 articles, then more than one package of articles is required to complete the testing. If the package contains 14 or more articles, then only one package of articles is required to perform the testing. If the package contains 4 or fewer articles then all articles in the package are measured and multiple packages are required to perform the measurement. Caliper readings should be taken 24 ± 1 hours after the article is removed from the package, unfolded and conditioned. Physical manipulation of product should be minimal and restricted only to necessary sample preparation.

Any elastic components of the article that prevent the article from being laid flat under the caliper foot are cut or removed. These may include leg cuffs or waistbands. Pant-type articles are opened or cut along the side seams as necessary. Apply sufficient tension to flatten out any folds/wrinkles. Care is taken to avoid touching and/or compressing the area of measurement.

### In-Bag Stack Height Test

The In-Bag stack height of a package of absorbent articles is determined as follows:
**Equipment:** A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams. Such a testing apparatus is for example illustrated on Fig. 19 of US2008/0312624A1.
**Test Procedure:** Absorbent article packages are equilibrated at 21 ± 2 °C and 50 ± 5% relative humidity prior to measurement. The horizontal sliding plate is raised and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

### Lateral Absorbent Zone Profile Ratio Test

This test can be used to assess the profiling of absorbent material in the transversal direction for the lateral absorbent zone 61, 63 and for a variety of channel shapes 26. In this method, the lateral absorbent zones of the core are sub-sectioned, cut apart, and weighted to determine relative presence of absorbent material. The core wrap is weighted along with the contained absorbent material for the purpose of the test.

The absorbent core can be extracted from a commercial article (e.g. a diaper) after manually removing the other components such as back ears, barrier cuffs, waist bands (if present), backsheet, topsheet and acquisition layer of acquisition/distribution system. Cryogenic freeze spray may be used sparingly to separate adhesive bonds if necessary.

On the extracted core, the lateral absorbent zone 61, 63 are identified and highlighted using a suitable marker. Each lateral absorbent zone extends transversally from a longitudinal channel 26a,b to the respective longitudinal edge 604, 606 of the absorbent material layer 60. Each lateral absorbent zone is labeled Left and Right respectively.

The marker is then used to draw the separating lines 67a, 67b, which are at all points equidistant between the respective channel and the longitudinal edge of the absorbent layer along the transverse direction, thus delimiting an inner region 66 and outer region 68 of the lateral absorbent zone, with the inner region and outer region having substantially the same area. When the channels and the longitudinal side edges of the absorbent layer are straight, the separating line is thus also straight (see e.g. Fig. 9), however the channels and the longitudinal side edges of the absorbent layer can also have more complex shape (e.g. as in Fig. 10), in which case the separating line can have a non-straight outline.

The lateral absorbent zones 67a, 67b on each side of the longitudinal centerline 80 are then carefully separated by cutting (using suitable means such as scissors or a rotary cutter) along the separating line 67a, 67b. The absorbent core is cut apart and subsequently handled to minimize any loss of absorbent material from excised portions.

The inner region 66a and outer region 68a for the first (Left) absorbent zone 61, and the inner region 66b and outer region 68b for the second (Right) absorbent zone 63, are likewise separated by cutting along the separating line 67a, 67b respectively. The core wrap is also cut at the same time as the inner and outer regions are isolated. Since the absorbent material is typically at least partially adhesively attached to the core wrap, the core wrap is included in the following measurements.

The weight of the inner region (Wi) and the weight of the outer region (Wo) are then measured for each lateral absorbent zone (Left and Right) using a laboratory scale with a precision of at least 0.01 g.

The procedure is repeated for at least 5 like-absorbent cores sampled from five like-articles randomly selected inside an absorbent article package, and the results averaged for each lateral absorbent zone and region, providing four average values (WiR, WoR, WiL, WoL).

The ratio of the weight of inner region and the outer region is then calculated by dividing the average value of the inner region Wi with the average value of the outer region WO for respectively the Left and Right lateral absorbent zones. The result is the Lateral Absorbent Zone Profile Ratio for each lateral absorbent zone (referred to as RaL for the Left lateral absorbent zone, and RaR for the Right lateral absorbent zone) and is reported to the nearest 0.01.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article (20) for personal hygiene comprising a longitudinal centerline (80) extending in a longitudinal direction (y) and a transversal centerline (90) extending in a transversal direction (x) perpendicular to the longitudinal direction, the absorbent article comprising:
a topsheet (22);
a backsheet(24);
an absorbent core (28) between the topsheet and the backsheet;
optionally an acquisition layer (52) between the topsheet and the absorbent core;
wherein the absorbent core (28) comprises:
- a core wrap (16, 18) comprising a top side (16) and a bottom side (18);
- an absorbent layer (60) between the top side (16) and the bottom side (18) of the core wrap, wherein the absorbent layer comprises an absorbent material comprising superabsorbent polymer particles optionally mixed with cellulose fibers, and the absorbent layer extends in the longitudinal and transversal directions, wherein the absorbent layer (60) has a periphery comprising a front edge (600), a back edge (602) and first longitudinal side edge (604) on one side of the longitudinal centerline (80) and a second longitudinal side edge (606) on the other side of the longitudinal centerline (80);
- a first and second longitudinally-extending channels (26a, 26b) within the absorbent layer, wherein the channels are substantially free of absorbent material and wherein the top side of the core wrap is attached to the bottom side of the core wrap through the channels, wherein the first channel (26a) and the second channel (26b) are symmetrically disposed on each side of the longitudinal centerline, wherein the first channel (26a) and the second channel (26b) can be partially or entirely the same channel or different channels;
- a first lateral absorbent zone (61) and a second lateral absorbent zone (63) extending respectively transversally from the first channel (26a) to the first longitudinal side edge (604), and from the second channel (26b) to the second longitudinal side edge (606);
wherein the first and second lateral absorbent zones are profiled in the transversal direction so that the first lateral absorbent zone and the second lateral absorbent zone each has a Lateral Absorbent Zone Profile Ratio of at least 1.40, as measured according to the Lateral Absorbent Zone Profile Ratio Test described herein.

2. The absorbent article according to claim 1, wherein each lateral absorbent zone has a Lateral Absorbent Zone Profile Ratio of from 1.40 to 3.00, as measured according to the Lateral Absorbent Zone Profile Ratio Test described herein.

3. The absorbent article according to any of the preceding claims, wherein the absorbent article further comprises:
- a central absorbent zone (62) disposed between the first channel (26a) and the second channel (26b).

4. The absorbent article according to the preceding claim, wherein the amount of absorbent material in the central absorbent zone (62) ranges from about 5% to about 25% of the total amount of absorbent material in the absorbent core, and the combined amount of absorbent material in the first and second lateral absorbent zones (61, 63) ranges from about 30% to 90% of the total amount of absorbent material in the absorbent core.

5. The absorbent article according to any of claims 3-4, wherein either the first and second channels are distinct, straight, and parallel to the longitudinal centerline, or wherein the first and second channels are at least partially distinct and inwardly curved towards the longitudinal centerline.

6. The absorbent article according to any of claims 3-5 , wherein the central absorbent zone (62) has a maximum width (D) which is of at least 10 mm.

7. The absorbent article according to any of the preceding claims, wherein the length (L3) of the first and second channels is at least 10 cm, as measured in the longitudinal direction.

8. The absorbent article according to any of the preceding claims, wherein the absorbent material consists of superabsorbent polymer particles and is free of cellulosic fibers, in particular wherein the superabsorbent polymer particles are immobilized within the core wrap by a microfibrous adhesive.

9. The absorbent article according to any of the preceding claims, wherein the absorbent core comprises an auxiliary glue (72) between the absorbent material (60) and at least one of the top side (16) or the bottom side (18) of the core wrap.

10. The absorbent article according to any of the preceding claims, wherein the auxiliary glue (72) at least partially adhesively immobilizes the absorbent material in the central absorbent zone (62) and in at least a portion of lateral absorbent zones (61, 63) disposed adjacent the channels.

11. The absorbent article according to any of the preceding claims, wherein the absorbent article comprises an acquisition layer (52) between the topsheet and the absorbent core, wherein the acquisition layer is narrower than the absorbent layer (60), in particular wherein the acquisition layer overlaps the central absorbent zone (62) of the absorbent layer, if present, but the acquisition layer overlaps only a portion of each of the first and second lateral absorbent zones disposed adjacent the first and second channels.

12. The absorbent article according to any of the preceding claims, further comprising a front absorbent zone (64) comprising absorbent material and disposed longitudinally outwardly of the channels towards the front edge of the core (280), and a back absorbent zone (65) comprising absorbent material and disposed longitudinally outwardly of the channels towards the back edge of the core (282).

13. An absorbent article (20) for personal hygiene comprising a longitudinal centerline (80) extending in a longitudinal direction (y) and a transversal centerline (90) extending in a transversal direction (x) perpendicular to the longitudinal direction, the absorbent article comprising:
a topsheet (22);
a backsheet (24);
an absorbent core (28) between the topsheet and the backsheet;
optionally an acquisition layer (52) between the topsheet and the absorbent core;
wherein the absorbent core comprises:
- a core wrap (16, 18) comprising a top side (16) and a bottom side (18);
- an absorbent layer (60) between the top side (16) and the bottom side (18) of the core wrap, wherein the absorbent layer comprises an absorbent material comprising superabsorbent polymer particles mixed with cellulose fibers, and the absorbent layer extends in the longitudinal and transversal directions, wherein the absorbent layer (60) has a periphery comprising a front edge (600), a back edge (602) and first longitudinal side edge (604) on one side of the longitudinal centerline (80) and a second longitudinal side edge (606) on the other side of the longitudinal centerline (80);
- a first and second longitudinally-extending channels (26a, 26b) within the absorbent layer, wherein the channels are substantially free of absorbent material and wherein the top side of the core wrap is attached to the bottom side of the core wrap through the channels, wherein the first channel (26a) and the second channel (26b) are symmetrically disposed on each side of the longitudinal centerline, wherein the first channel (26a) and the second channel (26b) can be partially or entirely the same channel or different channels;
- a first lateral absorbent zone (61) and a second lateral absorbent zone (63) extending respectively transversally from the first channel (26a) to the first longitudinal side edge (604), and from the second channel (26b) to the second longitudinal side edge (606);
wherein the first and second lateral absorbent zones are profiled in the transversal direction so that the basis weight of the absorbent material in each lateral absorbent zone is higher towards the respective channel than towards the respective longitudinal side edge;
wherein the first longitudinal side edge and the second longitudinal side edge of the absorbent layer are generally straight and parallel to the longitudinal direction.

14. A package comprising a plurality of absorbent articles according to any of the preceding claims.

15. An absorbent core comprising:
- a core wrap (16, 16') comprising a top side (288) and a bottom side (290);
- an absorbent layer (60) between the top side (16) and the bottom side (18) of the core wrap, wherein the absorbent layer comprises an absorbent material comprising superabsorbent polymer particles optionally mixed with cellulose fibers, and the absorbent layer extends in the longitudinal and transversal directions, wherein the absorbent layer (60) has a periphery comprising a front edge (600), a back edge (602) and first longitudinal side edge (604) on one side of the longitudinal centerline (80) and a second longitudinal side edge (606) on the other side of the longitudinal centerline (80);
- a first and second longitudinally-extending channels (26a, b) within the absorbent layer, wherein the channels are substantially free of absorbent material, wherein the top side of the core wrap is attached to the bottom side of the core wrap, and wherein the first channel (26a) is disposed on one side of the longitudinal centerline and the second channel (26b) on the other side of the longitudinal centerline;
- wherein the first and second channels each has a length (L') as measured in the longitudinal direction;
- a central absorbent zone (62) disposed between the first channel and the second channel; and
- a first lateral absorbent zone (61) and a second lateral absorbent zone (63) extending respectively transversally from the first channel (26a) to the first longitudinal edge, and from the second channel (26b) to the second longitudinal edge;
wherein the first and second lateral absorbent zones are profiled in the transversal direction so that the basis weight of the absorbent material in each lateral absorbent zone is higher towards the respective channel than towards the respective longitudinal side edge, so that the first lateral absorbent zone and the second lateral absorbent zone each has a Lateral Absorbent Zone Profile Ratio of at least 1.40, as measured with the Lateral Absorbent Zone Profile Ratio Test described herein.
